Europäisches Patentamt

European Patent Office

Office européen des brevets

(19)

(11) Publication number: 0 328 401

A1

(12) EUROPEAN PATENT APPLICATION

(21) Application number: 89301286.4

(22) Date of filing: 10.02.89

(51) Int. Cl.⁴: A 61 B 17/11

(30) Priority: 11.02.88 AU 6697/88

(43) Date of publication of application:
16.08.89 Bulletin 89/33

(84) Designated Contracting States: DE FR GB SE

(71) Applicant: UNISEARCH LIMITED
221-227 Anzac Parade
Kensington New South Wales, 2033 (AU)

(72) Inventor: Rogers Gregory James
198 Ellesmere Road, Gymea Bay
New South Wales, 2227 (AU)

Milthorpe Bruce Kenneth
28 Earl Street, Roseville
New South Wales, 2069, (AU)

Schindhelm Klaus
72 Francis Greenway Drive, Cherrybrook,
New South Wales, 2120, (AU)

(74) Representative: Stuart, Ian Alexander et al
MEWBURN ELLIS & CO. 2/3 Cursitor Street
London EC4A 1BQ (GB)

(54) Anastomosis augmentation device.

(57) For reinforcing a junction between a tendon or ligament and another (portion of) tendon or ligament or a graft or bioprosthesis, the junction or anastomosis is closely surrounded by an augmentation device. This comprises a tube woven from a biocompatible thread such that as the tube is shortened its internal diameter increases, and as it is lengthened, the diameter decreases. The tube may be loosely woven with both the warp and weft threads extending helically through the tube in opposite directions. It may be of slowly degrading polyester. The device preferably includes a shield which prevents adhesion of surrounding tissue following insertion of the augmentation device, suitably of bovine pericardium, xenograft fascias or resorbable polymeric membranes or weaves.

**Description**

**Anastomosis Augmentation Device**

The present invention relates to an anastomosis augmentation device and more particularly a device to augment an anastomosis (or junction) between a tendon or ligament on the one hand and another element such as a tendon, ligament, graft or bioprosthesis on the other.

Tendons are structural elements of the body which must support high tensile loads whilst gliding freely within the surrounding tendon sheath. When damaged or severed tendons are replaced or rejoined the resulting anastomosis (or junction) has been proven to be a point of continuing weakness. Hence, if a repaired or reconstructed tendon is to function normally the tendon/tendon, tendon/graft or tendon/prosthesis anastomosis must be strong and free from adhesions to surrounding tissues. In addition, in many situations such as hand surgery, the size (diameter) of the anastomosis is restricted and must not be excessive relative to the mid-section of the original native tendon.

Similarly ligaments are structural elements connecting the articular extremities of bones or supporting or restraining an organ. Unlike tendons they do not need to glide freely, but like tendons they repair slowly and support extensive tensile loads.

Traditionally the tendon/tendon or tendon/graft ends are sutured together using suture materials such as monofilament polypropylene (Prolene or Surgilene), braided polyester (Mersilene), Teflon impregnated braided polyester (Tevdek), nylon (Ethilon) or stainless steel. Common suture techniques such as Bunnel figure eight, Kessler grasping, Massen-Allen, interrupted sutures, Nicoladoni, Pulvertaft fish mount, End-weave and modifications of these produce anastomoses with initial ultimate tensile strengths ranging from 16 to 63N. These anastomosis strengths are an order of magnitude less than the strengths of the native tendon. These low 'time-zero' (immediately postoperative) anastomosis strengths and the possibility of rupture of anastomoses necessitates prolonged and complex postoperative immobilisaton regimes.

A recent study conducted at the Centre for Biomedical Engineering, University of New South Wales, Sydney, Australia illustrated the need for continued anastomosis augmentation especially for prosthetic tendon reconstruction. In the study ovine digital extensor tendons were replaced with either autogenous tendon or xenograft bioprosthesis. The xenograft anastomosis strengths had significantly increased from 30 N at time of operation to 100 N at 6 months. The 6 month anastomosis strength is approximately one quarter to a third of the strength of the original tendon. The xenograft anastomosis strengths did not significantly increase over the period from 6 months to one year. In comparison, the tendon/autograft anastomoses strengths were approximately half that of the initial tendon strength after 12 months implantation.

A similar study has been reported in which the achilles tendon of rabbits was reconstructed using a 15 mm segment of a tanned bovine bioprosthesis (Xenotech). From this study it was concluded that load transfer across the anastomosis was minimal as there was little or no host invasion of the bioprosthesis. In another study, Leghorn chicken profundus tendons were reconstructed using tanned kangaroo tail tendon. The strengths of a total of 14 tendon/xenograft/tendon complexes were measured at 'time-zero', 12 and 24 weeks. At each time point the samples failed at either the distal or proximal anastomoses.

These studies exhibit a need for both initial and continuing augmentation of tendon/tendon, tendon/graft or tendon/prosthesis anastomoses.

The present invention consists in an anastomoses augmentation device comprising a tube woven from a biocompatible thread such that as the tube is shortened so its internal diameter increases and vice versa, the tube being dimensioned so as to fit over and closely surround an anastomoses between a tendon or ligament on the one hand and another element selected from the group comprising a tendon, a ligament, a graft and a bioprosthesis on the other.

The tube is preferably loosely woven with both the warp and weft threads extending helically through the tube in opposite directions. Such an arrangement allows the tube to be shortened and radially expanded or lengthened and radially contracted. The size of the material used in the production of the augmentation device is a function of the desired proportion of an open weave, helix angle, number of the warp and web threads used and the acquired strength of the device. As would be readily understood by a person skilled in the art, the more strands spreading the load the better. However, the number of strands used will be limited by the desirability to keep a relatively open weave and the number of strands that can be readily woven into the device. At present it is preferred that the weave is such that it is approximately 50% or more open at a rest helix angle of 45°. Such an open weave will encourage fibrous encapsulation of the augmentation device. It is also presently preferred that the helix angle is approximately 45°.

In use the tube is placed over the anastomoses between two sections of tendon, a tendon and a graft or some other similar junction and if desired the end of the tube tied or sutured to the endogenous tendon or ligament and the element to which it is attached. As the tendon or ligament and the element in which it is attached are subjected to tension trying to draw them apart the tube will be stretched and will simultaneously contract more tightly gripping the tendon or ligament and the element respectively.

The augmentation device can be manufactured with a drawstring in each end of the device. The 2 drawstrings are used to provide initial attachment of the device to the underlying tendon, graft or prosthesis and are supplemented by suturing and later fibrous tissue ingrowth.

If the augmentation device is intended for permanent augmentation the tube and any sutures or ties connecting its ends to the tendon or other element are formed of nonabsorbable materials. If however, it is intended that the augmentation device is to provide support for the anastomosis for a limited term, say up to 2 years, the tube may be formed from materials having a slow resorption rates such as poly-L-lactide.

At present the augmentation device tested has been Dacron tube, due to its ready availability in appropriate dimensions. However, it is believed to be preferable that the augmentation device is composed of a long term (at least 6 months) resorbable fibre. Slowly degrading polyesters which would be suitable currently exist for other medical applications.

It is preferred that the weave of the augmentation device should be quite open to allow the device to easily and freely contract around the tendon or other element as the anastomosis is stretched. It is also desirable that the weave be sufficiently open to allow fibrous tissue to grow through the tube and onto the underlying tendon or other element.

In a preferred embodiment of the present invention when the augmentation device is applied to tendons, the device is provided with a shield which prevents adhesion of surrounding tissue following insertion of the augmentation device. This shield may be appropriately treated bovine pericardium, xenograft fascias or resorbable polymeric membranes (e.g. polylactic/polyglycolic acids).

It is to be understood that the augmentation device is adapted to augment an anastomosis between a tendon or ligament and any other suitable element. This other element may be another part of the subjects own tendon or ligament as where a tendon or ligament has been severed and is being regained. The other element may be a graft whether of the autograft, homograft or xenograft type. The element could alternatively be a prosthesis which is in turn attached to a bone, muscle or the like.

The augmentation device according to this invention allows controlled use of the tendon or ligament very soon after repair. This accelerates rehabilitation and reduces the formation of adhesions along the graft.

The intended application of the tendon augmentation device will govern its overall size. For example, repair or union of digital extensor tendons may require a device of approximately 15 mm length when expanded to a diameter of 7 to 8 mm prior to insertion. When the device is tensioned over the anastomosis its length will be in the order of 30 mm at a diameter of 3 mm.

A typical application for the tendon augmentation device would be to attach a xenograft bioprosthesis to a digital extensor tendon. The tendon would be exposed and prepared as would have been done previously. Twenty to 25 mm of free tendon should be left protruding from the tendon sheath to allow attachment of the tendon augmentation device. The xenograft should be inserted and the tendon augmentation device slipped over the end of the tendon (or xenograft) so that approximately 10 mm of the end of the tendon (or xenograft) remain visible.

The tendon and xenograft are joined using a Bunnel stitch and a 2 metric nonabsorbable, monofilament suture material (eg. polypropylene, Prolene, Ethicon). The tendon augmentation device is attached to the underlying tendon (or xenograft) at one end by tying the appropriate drawstring.

The device is then tensioned across the anastomosis and the remaining drawstring tied. Both ends of the augmentation device are then sutured to the underlying tendon (or xenograft), the operation site is closed in layers and the tendon immobilized.

Immobilization periods of the repaired or replaced tendon should be relatively short and early passive motion introduced to reduce the possibility of adhesions.

In order that the nature of the present invention may be more clearly understood preferred forms thereof will now be described with reference to the following examples.

Method used in Examples

The medical digital extension (DE) tendon in the left foreleg of four sheep was replaced by a 16-18cm length of partially-lysine-deaminated 2% glutaraldehyde (GA) kangaroo tail tendon (KTT). The removed portion of the medial DE tendon was used to replace a 14-16cm length of the lateral DE tendon in the same foreleg, thereby giving a xenograft and an autograft for study.

The four anastomoses were sutured using a Bunnel technique and 3/0 monofilament polypropylene (Prolene, 8976H, Ethicon) suture. In addition, an augmentation device composed of dacron was used to augment each anastomosis.

The sheep were clinically assessed prior to operation and then again at one, two and three months post operatively. The sheep were sacrificed at three months post implantation and the augmented tendon anastomoses were harvested and grossly examined. Either the distal or proximal anastomosis were assessed mechanically whilst the other was placed in 10% of buffered formulin for later histopathological examination.

The augmentation device (AD) was prepared using the following protocol:-
- Wear gloves to keep Dacron clean.
- Expand the Dacron weave (2mm size) over a glass mandrill (7 - 7.5mm glass pipette) and cut to 14mm lengths using the blunt side of a hot scalpel blade.
- Slip the cut sections of Dacron onto 30mm lengths of 4.8mm heat shrink.
- Suture a 2/0 Ethibond (braided polyester, Ethicon substitute for TiCron) draw-string in each end of the Dacron device so that there is approximately 60mm of excess length on each of the four free draw-string ends. The draw-string is placed as close to the end of the Dacron weave as possible (within 1mm from the end). The draw-string is to pass under and then over every second longitudinal rib in the Dacron weave.
- Place one black 2/0 silk pulling loop in one end near, but inside the draw-string (within 2mm from the end). The circumference of the loop is to be approximately 40mm (i.e. 20mm loop).

- The 2 protruding loops of the pulling loop are placed at approximately 90° to the free ends of the draw-strings.
- Wash the augmentation device with an equal number of 3mm plastic tubes using the following procedure:-
- 0.1% Triton in ultrasonic bath.
- 5 rinses in 0.2 micron filtered distilled water.
- 70% ethanol in 0.2 micron filtered distilled water.
- 5 rinses in 0.2 micron filtered distilled water.
- Air dry in laminar flow hood.
- Place ADs into the 3ml tubes.
- Seal AD and open tube in a suitable soft pack for ETO sterilisation.
- Terminally sterilise by ETO and allow at least one week for degassing.

Example 1

Four sheep were operated on according to the above method. Each sheep was monitored carefully during the post operative period and clinical assessments made at four weeks, eight weeks and twelve weeks. The animals were sacrificed at twelve weeks for mechanical, gross pathological and histological assessment.

Clinical Assessment

All four sheep showed an initial post operative drop inflexion to about 70 ± 15%. This recovered over the twelve week period to 97 ± 12%.

All augmented anastomoses palpated as intact, although the four week assessments of two of the xenograft anastomoses appeared slightly "hour glass" - indicative of some stretching of the anastomoses.

All the xenografts worked well to 12 weeks with palpable load transfer along the entire length. However, at 12 weeks only one autograft was felt to be "good".

Gross Pathology

All the Dacron ADs showed good fibrous in growth with plenty of visible blood vessels, however, a few were lightly adhered to the skin. In general, all xenograft mid sections were in goods condition with loose fibrous tissue attached. Only one very slight adhesion was noted. The anastomoses were also intact although some showed signs of being stretched. Fibrous tissue did not appear to effect the operation of the anastomoses.

Each of the autografts were unsuccessful, with only one being not significantly adhered to the surrounding tissues and some load transferred along the tendon by flexing the leg. The autograft's anatomoses showed massive adhesion to surrounding tissues, especially at the distal anastomosis. However, the autograft anastomoses were still very strong.

Mechanical Testing

- Xenograft mid-section strength remained reasonable high (300N).
- Xenograft anastomoses were stronger (approximately 20%) with augmentation (120 ± 20N cf. 95 ± 10N).
- Autograft mid-sections were very weak (120 ± 7N cf. approximately 400N previously).
- Autograft anastomoses were very strong (145 ± 40N cf. 85 ± 20N without augmentation).

Histology

The Xenograft material (nitrous/GA) was encapsulated with a thin closely associated fibrous capsule. A very small amount of ingrowth was visible at 12 weeks. This is characteristic of this material. The augmentation devices were well surrounded by fibrous tissue with a lot of vascularisation. No overt granulomatous reactions were observed.

The autograft were subject to varying degrees cellular activity with high cell density implying remodelling.

As shown by these results the AD allows early return to use after surgery due to rapid return to normal flexion. Also there were no overt ruptures of the anastomoses although this was common in previous study where no augmentation and long periods of immobolisation were used.

The AD also results in high anastomoses strengths for both xenografts and autografts for 12 weeks.

Example 2

As the initial study showed that the AD tended to adhere to surrounding tissues which resulted in weaker autografts due to resorption, a second study was initiated which included shielding the AD from surrounding tissue in an attempt to prevent adhesions. The protocol used in this study was identical to the initial study in other respects.

The shielding of the AD was achieved by using partially lysine-deaminated 2%-gluteraldehyde treated bovine pericardium.

Four sheep were operated on as described previously. Each sheep was monitored carefully during the post-operative period and clinical assessments made at four weeks, eight weeks and twelve weeks. Animals were sacrificed at twelve weeks for mechanical, gross pathological and histopathological assessment.

### Clinical Assessment

The assessment of clinical tendon function and palpation of an astomosis showed that the shielded AD performed better than the non-shielded devices. All anastomoses remained intact over the study period. All distal anastomoses could always be clearly identified and pulpated showing near normal force transfer at all points in the study. Xenograft and autograft proximal anastomoses were also judged to be intact throughout the study although it was occasionally difficult to distinguish the proximal autograft from xenograft anastomoses by palpation. Minimal fibrotic response was palpable at the anastomoses, which indicates that the uncontrolled fibrotic adhesions which occurred in the previous study were not present. At twelve weeks all implants were allowing good low transfer.

### Gross Pathology

All anastomoses were intact at sacrifice. All the Dacron ADs were well encapsulated with minimal fibrous tissue, the capsules were compact and generally did not interfere with the gliding function of the tendons. In one instance, the range motion of the joint was restricted by the proximal anastomoses not being able to pass through the carpal tunnel. In many instances the anastomoses were attached to surrounding connective tissue by loose connective tissue which did not restrict mobility.

All xenograft mid-sections were moving freely within their sheaths. No adhesions were noted. Only one autograft was adhered within the carpal joint capsule. This autograft had a slight reddish appearance but still appeared to have good force transfer through both anastomoses. All other autografts had free movement. The presence of loose connective tissue adhering to the graft but not affecting function was noted.

### Mechanical Testing

Table 1 summarises the ultimate tensile loads (UTL) obtained by the mechanical testing of the mid-sections and proximal anastomosis of the grafts.

## TABLE 1.

## Summary of UTLs(N) for Anastomoses, and Graft Mid-Sections.

|  | Xenograft Mid-section | Xenograft Proximal Anastomosis | Autograft Mid-section | Autograft Proximal Anastomosis |
|---|---|---|---|---|
| AD + Shield | 387±127 (n=4) | 141±39 (n=4) | 384±108 (n=4) | 136±18 (n=4) |
| AD only | 334±14 (n=3) | 120±20 (n=4) | 120±7 (n=4) | 145±40 (n=3) |
| No AD. | 389±105 (n=15) | 95±10 (n=6) | 379±69 (n=31) | 85±38 (n=36) |

Mid-section UTLs of xenografts and autografts were comparable (387 ± 127N cf. 384 ± 108N, n = 4). The mid-section strengths of xenografts were comparable with previous studies using the AD without shielding and no AD. However, there was a 200% increase in UTL of autografts mid-sections when compared to autograft reconstructions using only the AD.

An anastomosis strength of the proximal xenograft and autograft anastomoses were comparable (141 ± 39N cf. 136 ± 18N, n = 4). This is a 60% increase in autograft and 50% increase in xenograft anastomoses strength when compared with a previous study where no AD was used (see Table1).

### Histology

Xenograft material (nitrous/GA) was encapsulated with a thick closely associated fibrous capsule. Some radial cellular infiltration was observed at twelve weeks. This is characteristic of this material and indicates that gradual resorption with concommitent reorganisation was occurring.

The pericardial shield around the ADs was difficult to distinguish from the surrounding tissue. The shield was undergoing resorption. No inflammatory response was incited by the shield. The autografts appeared as normal tendons with the occasional blood vessel being present within the tendon.

The Dacron of the augmentation device was surrounded by mild inflammatory cells as is typical for woven Dacron. The autograft anastomoses was still highly cellular with some evidence of collagen reorientation along lines of stress. The xenograft anastomoses were highly cellular at the host tissue interface; only minimal cellular infiltration had occurred into the xenograft.

The AD allows early return to tendon use after surgery. No ruptures of anastomoses occurred, although this was common in previous study with no augmentation for longer period for mobilisation.

The shielded AD results in higher anastomoses strength when compared with no augmentation

In summary, the shielded AD has resulted in a stronger initial anastomoses for both xenograft and autograft applications. The device leads to early return to functional use of the reconstruction whilst lessening the incidents of rupture.

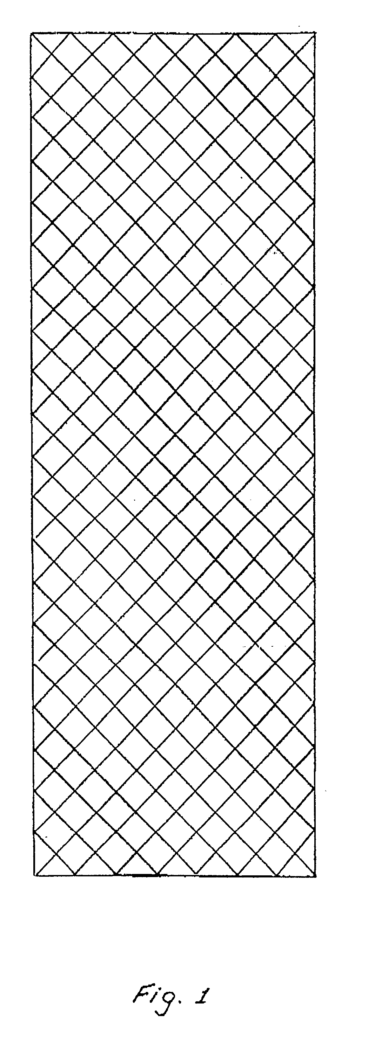

In order that the nature of the present invention may be more clearly understood a preferred form thereof will now be described with reference to the accompanying figure which shows diagrammatically an augmentation device according to the present invention, in which the device is formed of 7 warp and 7 weft threads with a helix angle of approximately 45°. The device is 7 cycles wide and 20 cycles high.

## Claims

1. An anastomosis augmentation device comprising a tube woven from a biocompatible thread such that as the tube is shortened its internal diameter increases and vice versa, the tube being dimensioned so as to fit over and closely surround an anastomosis between a tendon or ligament on the one hand and another element selected from
a tendon, a ligament, a graft and a bioprosthesis on the other.

2. An anastomosis augmentation device as claimed in claim 1 in which the augmentation device is provided with a shield which prevents adhesion of surrounding tissue following insertion of the augmentation device.

3. An anastomosis augmentation device as claimed in claim 2 in which the shield material is selected from
bovine pericardium, xenograft fascias and resorbable polymeric membranes or weaves.

4. An anastomosis augmentation device as claimed in any one of claims 1 to 3 in which the tube is loosely woven with both the warp and weft threads extending helically through the tube in opposite directions.

5. An anastomosis augmentation device as claimed in any one of claims 1 to 4 in which the warp and weft threads are slowly degrading polyesters.

6. An anastomosis augmentation device as claimed in any one of claims 1 to 5 in which the augmentation device is provided with a draw-string at each end of the device.

Fig. 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-4 469 101  (C.R. COLMAN et al.) <br> * Abstract; column 2, lines 40-47; column 3, lines 20-30,36-68; figure 1 * <br> --- | 1-6 | A 61 B  17/11 |
| Y | EP-A-0 122 744  (HOWMEDICA) <br> * Claim 1; page 10, line 16 - page 11, line 11; page 18, lines 9-17; figure 1 * <br> --- | 1-6 | |
| A | US-A-4 603 695  (Y. IKADA et al.) <br> * Column 3, lines 9-18; claims 3-5,16-18 * <br> --- | 2-3,5 | |
| A | DE-A-3 607 075  (AMERICAN HOSPITAL SUPPLY) <br> * Page 5, lines 4-13; page 8, lines 28-32; claims 2,7-8 * <br> --- | 2-3 | |
| A | EP-A-0 241 252  (JOHNSON & JOHNSON) <br> * Abstract * <br> --- | 5 | |
| A | WO-A-8 204 390  (K.Z. KURLAND) <br> * Whole document * <br> ----- | 1-6 | TECHNICAL FIELDS SEARCHED (Int. Cl.4) <br><br> A 61 B <br> A 61 F <br> A 61 L |

The present search report has been drawn up for all claims

| Place of search | Date of completion of the search | Examiner |
|---|---|---|
| THE HAGUE | 17-05-1989 | NICE P.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)